# EUROPEAN PATENT APPLICATION

(11) **EP 4 467 662 A1**
(43) Date of publication of application: **27.11.2024**
(21) Application number: 23175551.3
(22) Date of filing: 26.05.2023
(51) Int. Cl.: C12Q 1/6883

(54) **METHOD FOR PREDICTING THE TIME POINT OF CULTURE CONVERSION IN INDIVIDUALS**

(71) Applicant: Forschungszentrum Borstel, Leibniz Lungenzentrum, 23845 Borstel (DE)
(72) Inventor: Reimann, Maja, 22359 Hamburg (DE); Lange, Christoph, 23843 Bad Oldesloe (DE); Heyckendorf, Jan, 24105 Kiel (DE)
(74) Representative: Kröncke, Rolf

(57) **Abstract**

The present invention relates in a first aspect to a method for predicting the time point of culture conversion in individuals afflicted with mycobacterial infection comprising the steps of determining the expression level of genes in a sample obtained from said individuals, based thereon calculating a signature on the expression level of said genes followed by the calculation of the time point of culture conversion in said individuals. Moreover, the present invention relates to a computer-implemented method for predicting the time point of culture conversion computing the data of measured expression level of genes and based thereon predicting the time point of culture conversion. Moreover, the use of a test kit or kit of parts for determining the time point of culture conversion in an individual afflicted with mycobacterial infection is described whereby said kit comprises primers and/or probes for determining the expression level of the specified genes as well as instructions on how to use the same the method according to the present invention. Finally, the *in vitro* use of the genes as identified herein for predicting the time point of culture conversion in an individual is mentioned.

## Description

The present invention relates in a first aspect to a method for predicting the time point of culture conversion in individuals afflicted with mycobacterial infection comprising the steps of determining the expression level of genes in a sample obtained from said individuals, based thereon calculating a signature on the expression level of said genes followed by the calculation of the time point of culture conversion in said individuals. Moreover, the present invention relates to a computer-implemented method for predicting the time point of culture conversion computing the data of measured expression level of genes and based thereon predicting the time point of culture conversion. Moreover, the use of a test kit or kit of parts for determining the time point of culture conversion in an individual afflicted with mycobacterial infection is described whereby said kit comprises primers and/or probes for determining the expression level of the specified genes as well as instructions on how to use the same in the method according to the present invention. Finally, the *in vitro* use of the genes as identified herein for predicting the time point of culture conversion in an individual is mentioned.

### Prior Art

Tuberculosis (TB) remains a major global health threat with emerging *Mycobacterium tuberculosis* drug-resistance being particularly worrisome. This is one of the main reasons for death by bacterial infection. Treating *Mycobacterium tuberculosis* infection is time consuming, in particular in view of the slow cycle of division of *Mycobacterium tuberculosis* as well as the presence of resistance. Typically, treatment is carried out by standard therapy for six months according to the HRZE scheme, containing isoniazid, rifampicin, pyrazinamide and ethambutol for 2 months followed by 4 months of daily administered isoniazid and rifampicin, see https://www.who.int/publications/i/item/9789240050761, or an individual therapy regimen based on a determined resistance.

Monitoring of therapy in the beginning is typically conducted by obtaining data from cultivation of the bacteria and determining bacterial growth in said culture. These data on cultivation of the bacteria allows to make conclusion on the severity of the disease, the response to the applied therapy and, if necessary, triggers consequences on the therapy etc. The time point at which bacterial growth can no longer be detected represents the so-called point of culture conversion. It is generally accepted that this time point of culture conversion also represents the time point at which there is no longer a risk of infection to or by the patient or individual.

Although the data on cultivation and bacterial growth display high sensitivity on the status of the patient, several disadvantages are associated with the therapy monitoring based on the culture data. First, it may not be possible to obtain data on cultivation of *Mycobacterium tuberculosis* because e.g., sputum is not available (as for instance usually the case for infants and younger children). Second, the main disadvantage is the very slow bacterial growth, so that results on bacterial growth can only be obtained after several weeks of cultivation.

This means that a statement about the patient's conditions in terms of response to therapy as well as the severity of the disease and the time point at which there is no longer a risk of infection can only be made after several weeks.

Hence, there is a need to provide methods that allow therapy monitoring as well as determination the time point of the absence of risk of infection, eventually allowing prediction of the treatment duration or monitoring of treatment, determination of therapy response to a drug administered to said individual for the treatment of mycobacterial infection, or determination of the duration of hospitalization of said individual.

This is particularly true as the cultivation of cultures and the determination of the time point of culture conversation require appropriate laboratory space and trained personnel. However, since most persons suffering from TB do not live in industrialized countries, corresponding prerequisites are rarely available. In fact, recognized hot spot of tuberculosis infection are e.g., in Africa, Eastern Europe and Central Asia. Currently, about ten million people have TB with about 1.5 million TB patients die.

At present, there are no other surrogate markers for predicting treatment duration or monitoring treatment early in disease and/or therapy. However, there is an urgent need for early prognosis, particularly regarding culture conversion, severity of the disease and response to therapy.

Recently, the present inventors disclosed another method for treatment monitoring in patients afflicted with mycobacterial infections (WO 2021/165523 A1). This earlier method determines the expression level of certain genes to predict the days remaining until the end of therapy. Although this method can also be used (in part) for the monitoring of therapy response, it should be understood that culture conversion (i. e., the time when a patient is no longer infectious), and end of therapy (i. e., the time when therapy can be terminated) relate to different time points in the management of a mycobacterial infections.

Accordingly, there is a need for new methods that allow as early as possible to predict the time point of culture conversion in individuals, to predict the treatment duration, to monitor treatment, to determine the therapy response to drugs administered to said individual, and/or to determine the length (also identified as duration) of hospitalization of an individual afflicted with mycobacterial infection.

### Brief description of the present invention

In a first aspect, the present invention relates to a method for predicting the time point of culture conversion in an individual afflicted with mycobacterial infection comprising the steps of:
a) determining the expression level of the genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual;
b) calculating a signature based on the expression level of said genes determined in step a);
c) calculating the time point of culture conversion in said individual.

In another aspect of the present invention, the present invention relates to a computer-implemented method for predicting the time point of culture conversion in an individual afflicted with mycobacterial infection comprising the step of:
a) obtaining data of measured expression level of genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual;
b) computing the data of step a) and predicting the time of culture conversion,
c) optionally, further comprising the step of displaying the data of time of culture conversion on an output unit.

The present inventors recognized that the combination of genes and their identified expression level provides a suitable surrogate marker for predicting the time point of culture conversion, whereby the predicted time point of culture conversion may be in the past or in the future from the time of sampling. Namely, the outcome of the calculation is a time point of culture conversion, which marks e.g., the day of culture conversion accordingly.

In another aspect of the invention the present invention refers to the use of a test kit or kit of parts for determining the time point of culture conversion in an individual afflicted with mycobacterial infection, said kit comprising primers and/or probes for determining the expression level of genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual. In addition, the test kit comprises instructions of how to use the test kit or kit of parts in the method according to the present invention. That is, the kit or kit of parts is useful for determining the time point of culture conversion in an individual afflicted with tuberculosis.

Finally, the *in vitro* use of the genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L. and WWC2 in a sample obtained from said individual for predicting the time point of culture conversion in an individual afflicted with mycobacterial infection, in particular, wherein the mycobacterial infection is tuberculosis, by determining the expression level of said genes in said sample of the individual afflicted with mycobacterial infections is disclosed.

### Brief description of the drawings

Figure 1 shows a flow chart of the method according to the present invention.
Figure 2 shows the predicted days till culture conversion in the test data set.
Figure 3 provides the data of predicted days till culture conversion in the validation data set.
Figure 4 shows the comparison of signatures mentioned in table 1.
Figure 5 depicts the calibrated probabilities of the different signatures.

### Detailed description of the present invention

In a first aspect, the present invention relates to a method for predicting the time point of culture conversion in an individual afflicted with mycobacterial infection comprising the steps of:
a) determining the expression level of the genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual;
b) calculating a signature based on the expression level of said genes determined in step a);
c) calculating the time point of culture conversion in said individual.

The present inventors aim to provide a surrogate marker based on the expression level of the specified genes that allows early prediction of e.g., treatment duration and/or monitoring the therapy response. In particular, the present invention enables early determination of response to drugs administered to said individual for treatment of mycobacterial infection, and/or determination of the duration of hospitalization of said individual, e.g., the requirement of quarantine of the individual due to mycobacterial infection and the risk of contagion. The expression level of the genes results in a signature based thereon, whereby said signature allows to calculate the time point of culture conversion of said individual.

Culture conversion is a well-known and well-accepted term for an early surrogate marker for monitoring the success of therapy, at least in the initial phase of therapy as well as for determining the risk of contagion of individuals.

That is, based on the signature obtained from the expression level of the genes, it is possible to calculate the time point of culture conversion in the individuals accordingly. In this connection, it is noted that the calculated time point of culture conversion, e.g., expressed in days may be a positive number or a negative number. While the positive number indicates the number of days remaining until culture conversion, the negative number indicates the number of days that have elapsed since culture conversion. Namely, the result of the method according to the present invention is typically a number e.g., as expressed in days, indicating the time point of culture conversion.

The method is based on determining the expression level of the mentioned 29 genes. For example, in a first calculation step, a bacterial burden score (BBS) is generated, based on the following 12 genes: PVRL2, COL9A2, GBP3, CYB5R1, FCGR1B, KIR2DS4, FAM20A, ATXN7L1, CACNB4, NFIA, IGHA1, RPS6KA4. In an embodiment, the expression level of the mentioned 12 genes is weighted in the calculation of the BBS.

Moreover, a bacterial clearance score (BCS) may be determined, wherein the expression level of the following six genes is considered: TOMM40L, SLC12A1, CHGA, WWC2, CMTM3, NT5E. In addition, the BCS may include values regarding the BBS and the days under therapy. In a preferred embodiment, the expression level of said genes are weighted in the calculation of the BCS. Finally, the respective scores, namely the BBS and the BCS are combined with the expression level of the following 11 genes: SPSB1, SOWAHD, CNTNAP3B, RPL7A, ADORA3, SCARF1, CD83, CRY1, LINC00926, SNX22, MICAL3, in order to calculate the time point of culture conversion. Further, the calculation of the time point of culture conversion may include information on the days under therapy, and all the different values incorporated may be weighted accordingly. Based on the method according to the present invention, it is possible to calculate the time point of culture conversion based on the expression level of the respective genes, which thus represents a surrogate marker for culture conversion.

The present inventors have recognized that it is not necessary (if at all possible) to perform the time-consuming, weeks-long cultivation of the mycobacteria in order to determine the time point of culture conversion. Rather, using the method of the invention, this time point of culture conversion in an individual afflicted with mycobacterial infection can be conveniently and quickly calculated at any time point with high sensitivity and in very good agreement with information obtained by culture cultivation.

Hence, the present method makes it possible to shorten the time taken to establish a therapy response, so that a patient with a mycobacterial infection can, if necessary, be switched at an early stage to an alternative, more effective therapy. In addition, the duration of the risk of infection, and thus the quarantine and/or hospitalization required, can be accurately predicted. All this reduces both the burden on the patient and the costs to the healthcare system, enabling better and more effective patient care.

In another aspect, the method for predicting the time point of culture conversion in individuals afflicted with mycobacterial infection is associated with the determination of therapy response to drugs administered to said individuals for treating mycobacterial infection.

In another aspect, the method according to the present invention for predicting the time point of culture conversion allows the hospitalization of said individual to be terminated based on the surrogate markers for culture conversion according to the present invention. In particular, for individuals afflicted with mycobacterial infection, determination of the time point when quarantine can be terminated is very important, and the present invention allows to determine this time point with high accuracy.

Unless otherwise indicated, the terms "treatment monitoring" and "therapy monitoring" are used interchangeably herein. Namely, the above method for predicting the time point of culture conversion can be used for the treatment monitoring or therapy monitoring as well as for predicting the treatment duration. If a high number is calculated for the time to culture conversion using the method according to the invention, then this indicates that the remaining therapy duration is long and/or the treatment with the present therapy regimen is not very promising. In contrast, a low or even negative number indicates a short remaining treatment duration or even the end of treatment. Furthermore, a low or negative number is an indicator of treatment success if the treatment is monitored accordingly.

Furthermore, if a negative value is calculated using the method according to the present invention for determining the time point of culture conversion, this is an indicator e.g., that said patient can be discharged from hospital, since no further quarantine of the individual is required. On the other hand, in case of a positive value being determined, the individual must remain in the hospital and/or be quarantined or kept under quarantine.

In this connection, treatment monitoring may include the determination of the patient's response to drugs administered to said individual for treating the mycobacterial infection, which includes both different dosages as well as specific combinations of drugs.

In an embodiment of the present invention, the method for predicting the time point of culture conversion in an individual afflicted with mycobacterial infection allows to determine the antimycobacterial activity of a drug or a combination of drugs administered to said individual. Namely, the reduction in the time to culture conversion, determined using the method of the invention, can be used as a measure of the efficacy of a drug or a combination of drug for the treatment of a mycobacterial infection and allows the success of a treatment or the need to adjust the therapy regimen to be determined. Early determination of possible treatment failure also reduces the risk of adverse effects, possibly connected to prolonged and/or ineffective treatment with a less efficacious therapy regime.

This is also true for candidate drugs, in particular, candidate drugs tested in clinical phases, like clinical phase 2. In particular, based on the use of the surrogate marker to calculate the time to culture conversion as a bench mark for treatment success, it is possible to evaluate these drugs or candidate drugs in clinical phases with respect to their *in vivo* activity against drug sensitive, but also resistant and/or multi-resistant strains, respectively.

In another embodiment, the method according to the present invention allows to determine the risk of infection for third parties. If, the result obtained with the method according to the invention indicates that the time of culture conversion has already passed, the risk of contagion, i.e. infection for third parties posed by the tested person, is low or non-existent.

Further, in an embodiment of the present invention, the method for predicting the time point of culture conversion in an individual afflicted with mycobacterial infection comprises determining the expression level of the genes in a sample selected from sputum, blood including whole blood, plasma and cell. In a preferred embodiment, the sample obtained from the individual is whole blood. In contrast to the prior art methods, which describe the determination of the time point of culture conversion using sputum samples, the present method is particularly useful for the determination of the time point of culture conversion from whole blood.

It is well-known in the art that sputum is not available from all patients, which is particularly true for infants and children. Thus, while the production of the sputum and, hence, the informative value of sputum-based methods is restricted to the availability of sputum samples, the method according to the invention, using the determination of the expression level of the referenced genes from whole blood, remains stable, making it possible to determine these biomarkers easily and reproducibly over time.

The method according to the present invention enables personalized therapy and individual treatment monitoring as well as well the prediction of the duration of hospitalization, treatment etc. The method according to the present invention as well as the underlying model allow a fast and reliable prediction of the time point of culture conversion, especially without the delay caused by the cultivation of slow-growing mycobacteria. In particular, as mentioned above, the conventional culture-based method usually takes several weeks before an assessment on the time point of culture conversion can be made.

In particular, when whole blood obtained from the individual is used as a sample, it is possible to conduct the method according to the present invention as point of care method for the management of the tuberculosis infection, which also applies to infection with MDR and XDR strains. It is particularly helpful, when individuals are quarantined or hospitalized to control the circumstances of their treatment and accommodation.

Of note, the present inventors disclosed recently another method for treatment monitoring in patients afflicted with mycobacterial infections (WO 2021/165523 A1). This earlier method determines the expression level of certain genes to predict the days remaining until the end of therapy. Although this method can also be used (in part) for the monitoring of therapy response, it should be understood that culture conversion (i. e., the time when a patient is no longer infectious), and end of therapy (i.e., the time when therapy can be terminated) relate to different time points in the management of a mycobacterial infections. Accordingly, the earlier method and the method of the present invention should be considered complementary. They provide different, clinically relevant information and therefore use different sets of genes and scores.

In an embodiment of the present invention, gene expression is determined based on the level of said genes present in the sample. Namely, in an embodiment, the transcription level of said genes is determined based on RNA expression, for example, RNA expression is determined in whole blood of patients.

In another embodiment, the method of the present invention comprises a step of amplifying nucleic acids, in particular, RNA or cDNA, obtained from said RNA after reverse transcription. Both, amplification, and reverse transcription, are carried out by methods known to the person skilled in the art.

An advantage of the method according to the present invention is that the method is independent of the pathogen and thus the used surrogate markers are also independent of the pathogen. In contrast, currently applied conventional methods require cultivation of the mycobacterial pathogen(s), which may be influenced by different parameters, including (but not limited to) the culture conditions, the culture media and/or the handling of the culture. Difficulties in cultivation may be detected only after weeks, while the method according to the present invention allows to repeat the tests immediately to determine the time point of culture conversion accordingly.

In another embodiment, the method of the present invention is used to predict the time point of culture conversion in an individual afflicted with mycobacterial infection, wherein the mycobacterial infection is caused by a multidrug resistant (MDR-TB) or extremely drug resistant (XDR-TB) strain. For MDR- and XDR-TB, it is particularly important to monitor treatment and determine therapy response to the drugs administered to the individual to treat the infection. Only this way, it is possible to monitor therapy response, and adjust the treatment strategy if it is recognized that the time point of culture conversion will occur very late or if no change in the predicted time point of culture conversion is observed during treatment.

In another embodiment of the present invention, the method according to the present invention is suitable for evaluating candidate drugs, in particular, candidate drugs being tested in clinical phase 2, with respect to their efficacy and efficiency. For example, during clinical phases in drug testing, drug approval and drug registration, early identification of the suitability of candidate drugs is helpful.

Further, the present invention relates to a computer-implemented method for predicting the time point of culture conversion in an individual afflicted with mycobacterial infection comprising the steps of:
a) obtaining data of measured expression level of genes as defined herein from a sample of an individual;
b) computing the data of step a) and predicting the time of culture conversion,
c) optionally, further comprising the step of displaying the data of time of culture conversion on an output unit.

In step b) the data may be computed by comparing the data or the scores of the individual to be analyzed with scores, signatures, or expression levels of said genes obtained from a data base or obtained as a measure to levels of cut off controls. However, the computing of the data does not require comparison with references but may be calculated by known models suitable for classification. For example, general linear regression model (GLM), naive bayes, bayesian belief network, multinomial naive bayes, gaussion naive bayes, bayesian network, averaged one-dependence estimators, logistic regression model, elastic net model, classification and regression trees (CART), iterative dichotomiser 3, C4.5, C5, M5 chi squared automatic interaction detection, conditional decision tree, decision stump, support vector machines (svm), hopfield network, per-ceptron, multilayer perceptrons, radial basis function, stochastic gradient descent, back propagation, k-nearest networks (kNN), k-means, k-medians, hierachical clus-tering, expectation maximisation, lasso regression, ridge regression, least angle regression, eclant algorithm, a priori algorithm, gradient boosting algorithms, learning vector quantization, self-organization map, locally weighted learning, convolutional neural networks, stacked auto-encoders, recurrent neural network, long short term memory network, deep belief network, deep bolzmann machine, PCA, Principal component regression, projection pursuit, PLSR, sammon mapping, multidimen-sional scaling, linear discriminant analysis, mixture discriminant analysis, flexible disc. analys., bagging, boosting, adaboost, blending, gradient boosted regression tree, stacking, genetic optimization algorithm.

For example, the computer-implemented method, designated for obtaining the data of measured expression level, comprises the classification with the Random Forest (RF) classifications with x-fold iterations, for example 1,000-fold iterations, like 3,000-fold iterations, e. g., 5,000-fold iterations. RF is applied particularly for validation as calibrated probabilities. Further, the computer-implemented method comprises GLM for sub-model and model to predict the remaining time of culture conversion.

The method runs on a suitable data processing unit. Said data processing unit may contain an output unit that allows to display the results, in particular, the predicted time point of culture conversion. The data processing unit may be connected to a data base storage unit or a data base retrieval unit that allows comparison of the data of the measured expression level of the genes according to the present invention, e.g., in form of a score or signature as described herein, with a score and/or signature and/or expression level of the genes obtained from other individuals and/or cohorts of individuals.

Further, the present invention relates to a computer-readable medium or computer program product having computer-executable instructions for performing the steps of the method according to the present invention, including the computer-implemented method as described herein.

In a further aspect, the present invention relates to the use of a test kit or kit of parts for determining the time point of culture conversion in an individual afflicted with mycobacterial infection, said kit comprising primers and/or probes for determining the expression level of genes as identified herein and instructions, how to use the test kit or kit of parts for a method according to the present invention. The skilled person is well aware of suitable means for determining the expression level of a gene. Said means include components suitable for amplification of nucleic acids including RNA and DNA. For example, in an embodiment of the present invention, the test kit or kit of parts includes an amplification kit for amplification of nucleic acids.

Moreover, the test kit or kit of parts may contain components for prior purification or treatment of the sample obtained from said individual to be tested. Such compounds or components include means for preparing and providing RNA for further use. Such means also include enzymes and suitable buffer systems for isolating and/or purifying the RNA used, while separating other cellular components.

Further, the test kit or kit of parts according to the present invention includes means for detecting the amplification products or the expression level of said genes generally. Said means may include suitable probes, which may be labelled with suitable labels or marker molecules. Alternatively, the amplification products may be labelled based on using labelled modified nucleic acid molecules. The skilled person is well aware of suitable methods for performing amplification labelling accordingly, e.g., using expression microarrays, next generation sequencing etc.

Further, an array, such as a microarray or next generation sequencing, is provided, comprising means for detecting the expression of the genes ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual. In an embodiment, the present invention relates to the use of the test kit or the kit of parts or the array as described in a method according to the present invention.

Moreover, the present invention relates to the *in vitro* use of the genes as defined herein for predicting the time point of culture conversion in an individual afflicted by mycobacterial infection by determining the expression level of said genes in a sample of an individual afflicted with mycobacterial infection, in particular, tuberculosis.

The present invention will be described further by way of examples, without limiting the same thereto.

### Material and Methods

Between 2013 and 2019, a total of five cohorts consisting of antibiotic-sensitive (DS) and multidrug-resistant (MDR) tuberculosis (TB) patients were recruited in Germany - the German Identification Cohorts (DS-GIC & MDR-GIC) (training cohort), German Validation Cohorts (DS-GVC & MDR-GVC) (training coherent and test cohort) and Second German Validation Cohort (SGVC; both DS and MDR-TB patients) (validation cohort).

The study visits of the German cohorts included a clinical assessment and blood collection for whole blood RNA measurements from PAXgene tubes (Qiagen^{®}, Venlo, The Netherlands). Study visits ideally occurred before treatment initiation, after 14 days of therapy, at the time of smear conversion, and after culture conversion at 6 months and/or at the end of therapy for patients with drug-sensitive TB (DS-TB) and additionally after 10, 15, and 20 months of therapy for patients with MDR-TB. An additional period of one year after the end of therapy was further available for some patients. Sputum samples submitted by study participants were evaluated by smear microscopy and culture at the National Reference Center for Mycobacteria at the Borstel Research Center. Anti-tuberculosis therapy regimens were based on comprehensive drug sensitivity testing and were in accordance with current treatment recommendations.

Whole blood RNA isolation from PAXgene (Qiagen^{®}, Venlo, The Netherlands) was performed according to the manufacturer's instructions and stored at -80 °C until RNA isolation using the PAXgene Blood RNA Isolation Kit (Qiagen^{®}, Venlo, The Netherlands). Aliquots of the isolated RNA were used for quality control to analyze RNA integrity using the RNA Nano 6000 kit on an Agilent Bioanalyzer (Agilent^{®}, Böblingen, Germany) according to the manufacturer's instructions. In case of insufficient RNA integrity number (RIN) as a measure of sample quality and number, or if there were signs of degradation, samples were excluded from further analysis.

Total RNA was used for reverse transcription and subsequent Cy3 labeling using the Low-Input Quick Amp Labeling Kit (Agilent^{®}, Böblingen, Germany) according to the One-Color Microarray-Based Gene Expression Analysis Protocol version 6.9.1 (Agilent^{®}, Böblingen, Germany) with RNA spike-in controls. 1650ng Cy3-labelled cRNA were hybridized to human 4x44K V2 gene expression microarrays according to the manufacturer's instructions. Arrays were scanned using a SureScan microarray scanner (Agilent^{®}, Böblingen, Germany) at a resolution of 5 µm.

Raw expression data from scanned microarray slides were extracted from Tiff files using Feature Extraction software version 11 (Agilent^{®}, Böblingen, Germany). The raw data files were imported into Agilent GeneSpring software version 13 (Agilent^{®}, Böblingen, Germany). Percentile shift was used as the normalization method with the 75^{th} percentile as the target, and baseline transformation was applied to the median of all samples. Quality control was performed before data analysis, and compromised probes were removed from further analysis. The term "normalized expression" as used herein refers to Log2-transformed expression values.

Three additional external cohorts were obtained from the anTBiotic and ClickTB studies. These are cohorts from South Africa for which RNAseq was chosen as the extraction method instead of Agilent technology. All three cohorts were tested in a period of 14 days after the start of therapy with different therapy regimens. The created zipped fastq files were first unzipped and then an initial quality check was performed. The fastQC tool can be used to check the quality of pro-base sequences, GC content, and base content in addition to basic statistics. The reports were compiled into one report using MultiQC to quickly identify outliers or problematic samples and reanalyzed as necessary. Subsequently, the samples were cleaned up using cutadapt. In this process, unwanted sequences and adapters can be removed, which increases the quality of the data. In addition, this is a prerequisite for the following step. Fastq files are first converted into bam files. The complete sequence chain can be aligned to the human genome, allowing the gene sequences to be identified. The software tool hisat2 was used for this purpose.

After that, another quality control is necessary to check if the trimming, alignment and mapping was done correctly. This second quality control was performed with deeptools. After this quality control revealed no abnormalities, the individual bam files could be combined into a single data set and linked to the patient data. This count matrix was created using featurecounts and Rsubreads and formed the starting point for the analysis.

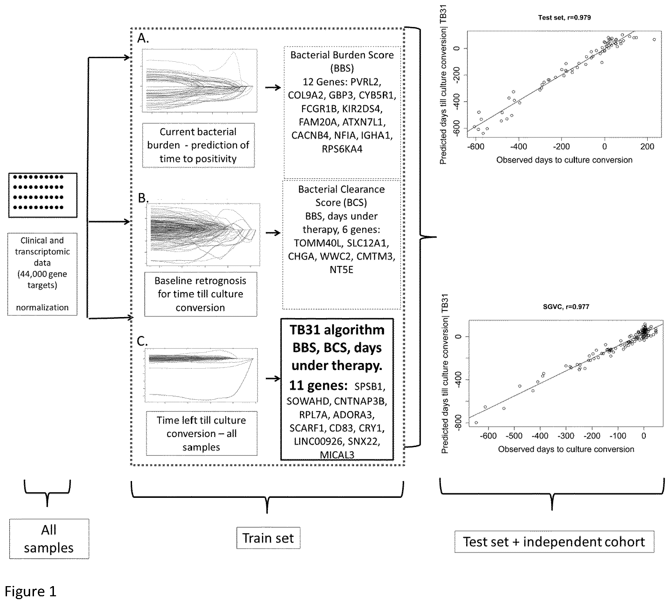

Modelling of the microarray data was performed with the software R (version 4.0.1) using the packages limma, glmnet, MASS, among others. The datasets of the German cohorts DS-GIC, DS-GVC, MDR-GIC, and MDR-GVC were divided into a training and a test dataset according to a 70:30 ratio, and SGVC served as an independent, external cohort (validation cohort). Genetic information was normalized using z-score within the three datasets. Figure 1 shows the modelled process and associated evaluation in the microarray datasets. This is a three-step process. In the first step, the "Bacterial Burden Score (BBS)" was modelled. For this, LASSO regression was used to identify genes that are important for predicting time to culture positivity (TTP). After further gene reduction steps, a model consisting of 11 genes was created to classify the current bacterial load by training the model on TTP.

The second step is a retro prediction for time to culture conversion at therapy start. For this, in addition to genes identified by LASSO procedure and reduced to 7, the days under therapy and the calculated BBS value were also included in the final model for the "Bacterial Clearance Score (BCS)".

In the final step, genes were identified that correlated with the days remaining until culture conversion at the time of blood collection. These genes, the days on therapy, the BBS, and the BCS were subjected to further variable reduction steps to produce the final model for TB31, which predicts the time remaining until culture conversion at the time of blood collection.

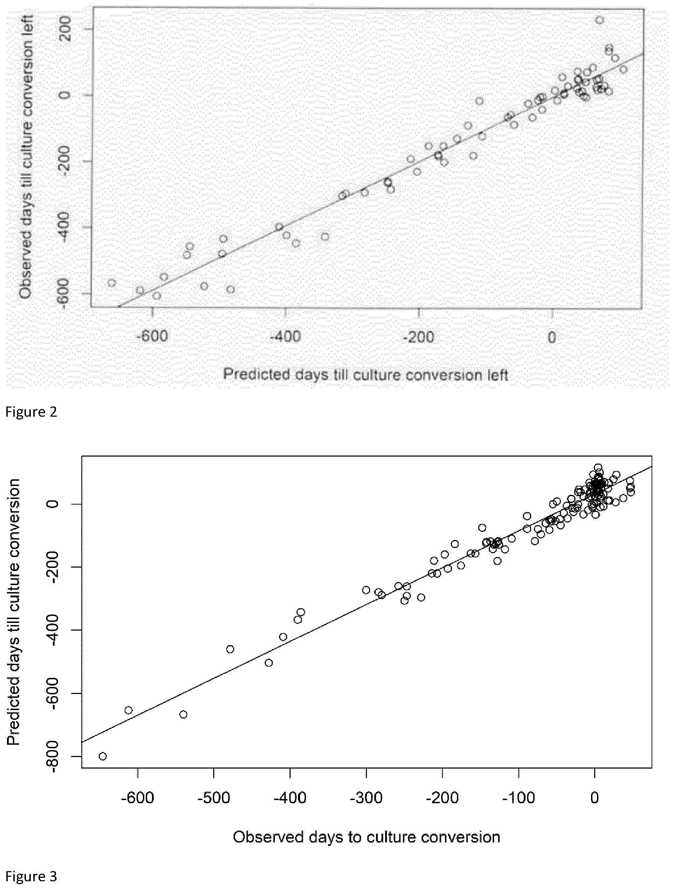

After the model was defined in the training data set and achieved a coefficient of determination of R²=0.98, the algorithm was first applied to the test data set. Here, a correlation coefficient of r=0.98 was found between the observed and remaining days to culture conversion (Figure 1, Figure 2). The difference between the observed and calculated remaining time to culture conversion was a median of 1.7 days with an interquartile range (IQR) of -4.9 - 11.6. The independent validation cohort SGVC also showed the same performance with r=0.98 (Figure1, Figure 3).

In addition to the high correlation between observed and calculated days remaining until culture conversion, plausible results regarding existing concepts of personalized medicine also emerged. For example, TB22, see below, did not indicate an end of therapy for any patient at any time point for which TB31 calculated a positive (=in the future) remaining time until culture conversion. Also, with respect to categorization into immune endotypes at therapy initiation, which provide information on whether a patient's immune system can respond inadequately to infection, showed that the calculated remaining time to culture conversion was significantly longer for patients with inadequate immune response.

In addition to clinical plausibility, the superiority of the TB31 algorithm over other previously described signatures, see Table 1, was tested to verify the added value of TB31.

**Table 1: Evaluated Signatures**

| Signatur | Original aim | Number of genes | R² | Correlation coefficient |
|---|---|---|---|---|
| TB31 according to the present invention | Prediction of time left till culture conversion | 29 | 0.982 | 0.98 |
| TB22 | Current on-time determination of the end of therapy at the time of measurement[1] | 22 | 0.173 | 0.66 |
| Anderson | Diagnosis in children[2] | 44 | 0.276 | 0.38 |
| Berry | Diagnosis [3] | 83 | 0.348 | 0.44 |
| Blankley | Diagnosis [4] | 4 | 0.187 | 0.60 |
| Kaforou | Diagnosis [5] | 27 | 0.213 | 0.49 |
| Kaforou | Diagnosis [5] | 44 | 0.323 | 0.214 |
| Kaforou | Diagnosis [5] | 53 | 0.286 | -0.01 |
| Laux da Costa | Diagnosis [6] | 5 | 0.187 | 0.59 |
| Maertzdorf | Diagnosis [7] | 3 | 0.137 | 0.50 |
| RISK6 | Prediction latent to active TB [8] | 6 | 0.166 | 0.56 |
| Sambaray | Diagnosis / current on-time therapy response [9] | 10 | 0.104 | 0.38 |
| Singhania | Diagnosis [10] | 20 | 0.201 | 0.54 |
| Suliman | Prediction latent to active TB [11] | 4 | 0.200 | 0.43 |
| Sutherland | Diagnosis [12] | 4 | 0.008 | 0.39 |
| Sweeney | Diagnosis, treatment response, disease severity [13] | 3 | 0.169 | 0.61 |
| Thompson | Prediction treatment outcome [14] | 9 | 0.167 | 0.51 |
| Thompson | Prediction treatment outcome [14] | 16 | 0.069 | 0.39 |
| Thomspon | Prediction treatment outcome [14] | 32 | 0.248 | 0.41 |
| Zak | Prediction latent to active TB [15] | 16 | 0.229 | 0.47 |

| | | | | |
|---|---|---|---|---|
| 1. Heyckendorf, J., et al., Eur Respir J, 2021. 58(3). 2. Anderson, S.T., et al., N Engl J Med, 2014. 370(18): p. 1712-1723. 3. Berry, M.P., et al., Nature, 2010. 466(7309): p. 973-7. 4. Blankley, S., et al., PLoS One, 2016. 11(10): p. e0162220. 5. Kaforou, M., et al., PLoS Med, 2013. 10(10): p. e1001538. 6. Laux da Costa, L., et al., Tuberculosis (Edinb), 2015. 95(4): p. 421-5. 7. Maertzdorf, J., et al., EMBO Mol Med, 2016. 8(2): p. 86-95. 8. Penn-Nicholson, A., et al., Sci Rep, 2020. 10(1): p. 8629. 9. Sambarey, A., et al.,. NPJ Syst Biol Appl, 2017. 3: p. 4. 10. Singhania, A., et al., Nat Commun, 2018. 9(1): p. 2308. 11. Suliman, S., et al., Am J Respir Crit Care Med, 2018. 197(9): p. 1198-1208. 12. Sutherland, J.S., et al., Clin Microbiol Infect, 2014. 20(4): p. O230-8. 13. Warsinske, H.C., et al., JAMA Netw Open, 2018. 1(6): p. e183779. 14. Thompson, E.G., et al., Tuberculosis (Edinb), 2017. 107: p. 48-58. 15. Zak, D.E., et al., Lancet, 2016. 387(10035): p. 2312-2322. | | | | |

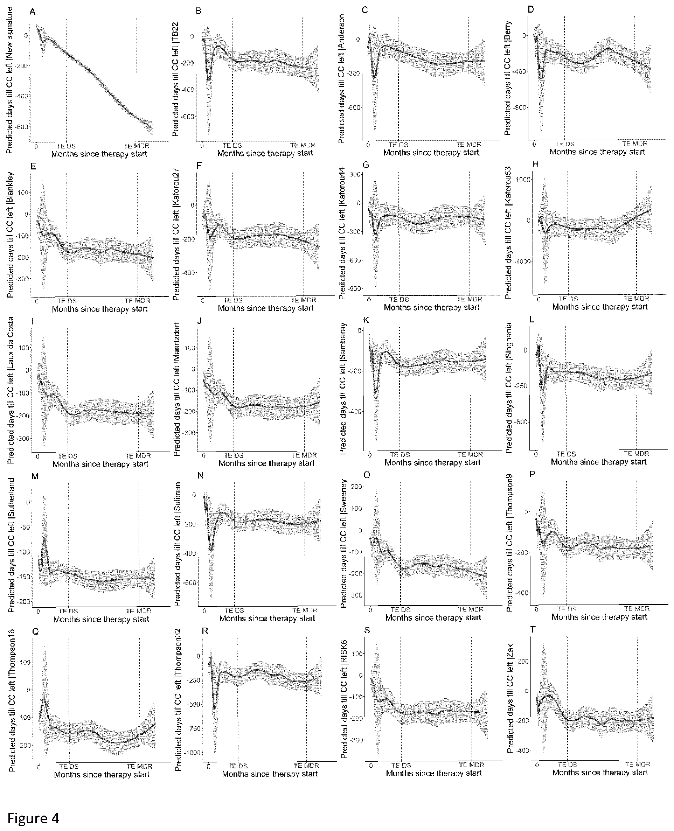

It was found that TB31 is the only signature that showed a stringent progression of prediction over the remaining time to culture conversion (Figure 4). Also, the coefficient of determination R² as well as the correlation coefficient r for the correlation between observed and calculated time remaining until culture conversion are the best (Table 1).

Furthermore, TB31 also showed the highest plausibility from another mathematical point of view. First, the remaining time until culture conversion was transformed into a binary concept classifying the time points "before culture conversion" and "after culture conversion" and the algorithms were compared with respect to their performance in random forest models. TB31 has the highest area under the curve (AUC) and the lowest out-of-bag (OOB) error (Table 2).

**Table 2**

| Signature | Number of genes | AUC | Out of bag error |
|---|---|---|---|
| TB31 | 29 | 0.97 (0.92 - 1) | 7.2% |
| TB22 | 22 | 0.83 (0.73 - 0.93) | 27.1% |
| Anderson | 44 | 0.82 (0.71 - 0.93) | 26.0 |
| Berry | 83 | 0.84 (0.73 - 0.94) | 29.8 |
| Blankley | 4 | 0.81 (0.70 - 0.93) | 34.3 |
| Kaforou | 27 | 0.82 (0.72 - 0.92) | 27.6 |
| Kaforou | 44 | 0.86 (0.77 - 0.95) | 24.3 |
| Kaforou | 53 | 0.88 (0.80 - 0.96) | 22.1 |
| Laux da Costa | 5 | 0.83 (0.73 - 0.93) | 33.7 |
| Maertzdorf | 3 | 0.78 (0.66 - 0.90) | 37.6 |
| RISK6 | 6 | 0.85 (0.76 - 0.94) | 29.3 |
| Sambaray | 10 | 0.79 (0.68 - 0.90) | 25.4 |
| Singhania | 20 | 0.83 (0.73 - 0.93) | 26.5 |
| Suliman | 4 | 0.85 (0.76 - 0.94) | 39.8 |
| Sutherland | 4 | 0.73 (0.60 - 0.85) | 40.3 |
| Sweeney | 3 | 0.79 (0.68 - 0.91) | 34.8 |
| Thompson | 9 | 0.84 (0.74 - 0.93) | 30.9 |
| Thompson | 16 | 0.76 (0.64 - 0.87) | 33.2 |
| Thomspon | 32 | 0.74 (0.61 - 0.86) | 30.4 |
| Zak | 16 | 0.79 (0.67 - 0.92) | 28.7 |

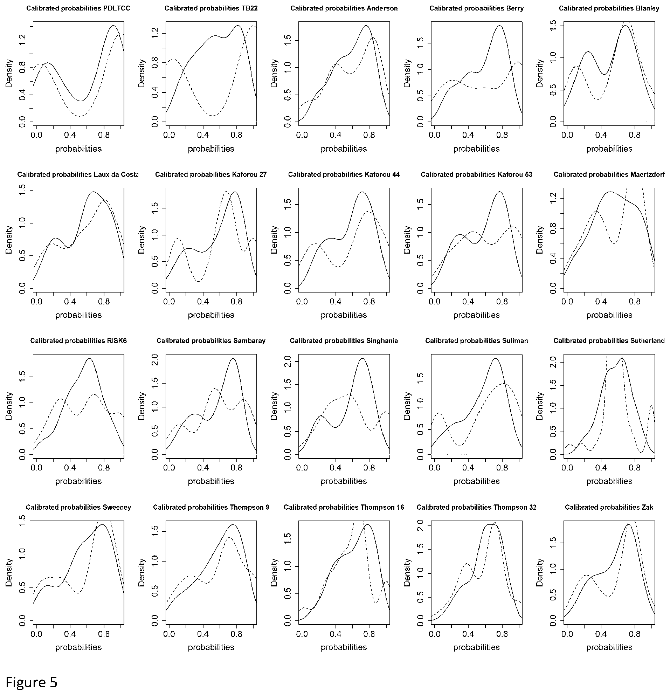

The calibration curves shown in Figure 5 compare how well the probabilistic predictions of a binary classifier are calibrated. Here, the actual frequency of positive labelling (continuous line) is plotted against the predicted probability (dashed line). The more similar the slopes of the two curves are, the better the model fits the actual prediction of the classification mentioned here to predict the true probability of the event.

## Claims

1. A method for predicting the time point of culture conversion in individuals afflicted with mycobacterial infection comprising the steps of
a) determining the expression level of the genes: ADORA3, ATXN7L1, CACNB4, CD83. CHGA, CMTM3, CNTNAP3B, COL9A2, CRY1, CYB5R1, FAM20A, FCGR1B, GBP3, IGHA1, KIR2DS4, LINC00926, MICAL3, NFIA, NT5E, PVRL2, RPL7A, RPS6KA4, SCARF1, SLC12A1, SNX22, SOWAHD, SPSB1, TOMM40L, and WWC2 in a sample obtained from said individual;
b) calculating a signature based on the expression level of said genes determined in step a);
c) calculating the time point of culture conversion in said individual.

2. The method for predicting the time point of culture conversion in an individual afflicted with mycobacterial infection according to claim 1 that allows i) prediction of treatment duration, ii.) treatment monitoring, iii) determination of therapy response to drugs administered to said individual for treating mycobacterial infection, and/or iv) determination of the duration of hospitalization of said individual, like determination of the required duration of quarantine of said individual due to the mycobacterial infection.

3. The method for predicting the time point of culture conversion in an individual afflicted with mycobacterial infection according to claim 1, for assessing the mycobacterial activity of an antimycobacterial drugs or drug candidate or a combination of drugs or drug candidates administered to said individual.

4. The method for predicting the time point of culture conversion in an individual afflicted with mycobacterial infection according to any one of the preceding claims for determining risk of contagion by said individual.

5. The method for predicting the time point of culture conversion in an individual afflicted by mycobacterial infection according to any one of the preceding claims wherein the expression level of the genes is determined in a sample selected from sputum, blood including whole blood, plasma and cell, preferably, the sample obtained from said individual is whole blood.

6. The method for predicting the time point of culture conversion in an individual afflicted by mycobacterial infection according to any one of the preceding claims wherein the expression of genes is determined based on RNA present in said sample.

7. The method for predicting the time point of culture conversion in an individual afflicted with mycobacterial infection according to any one of the preceding claims wherein the mycobacterial infection is tuberculosis, in particular, multidrug resistant tuberculosis.

8. The method for predicting the time point of culture conversion in an individual afflicted with mycobacterial infection according to any one of the preceding claims wherein the drug administered is a candidate drug, in particular, a candidate drug tested in clinical phase2.

9. A computer implemented method for predicting the time point of culture conversion in an individual afflicted by mycobacterial infection comprising the steps of:
a) obtaining data of measured expression level of genes as defined in claim 1 from a sample of an individual;
b) computing the data of step a) and predicting the time of culture conversion,
c) optionally, further comprising the step of displaying the data of time of culture conversion on an output unit.

10. Computer-readable medium or computer program product having computer-executable instructions for preparing the steps as identified in any one of claims 1 to 9.

11. The use of a test kit or kit of parts for determining the time point of culture conversion in an individual afflicted with mycobacterial infection, said kit comprising primers and/or probes for determining the expression levels of genes as identified in claim 1 and instructions of how to use the test kit or kit of parts for a method according to any one of claims 1 to 9.

12. The use of a test kit or kit of parts according to claim 11, said kit or kit of parts includes an amplification kit for amplification of nucleic acids.

13. The use of a test kit or kit of parts according to claim 11 or 12 for determining the time point of culture conversion in an individual afflicted with tuberculosis.

14. The *in vitro* use of the genes as defined in claim 1 for predicting the time point of culture conversion in an individual afflicted by mycobacterial infection by determining the expression level of said genes in a sample of an individual afflicted with mycobacterial infection, in particular, tuberculosis.
